# EUROPÄISCHE PATENTSCHRIFT

(11) **EP 1 157 020 B1**
(45) Veröffentlichungstag und Bekanntmachung des Hinweises auf die Patenterteilung: **25.09.2002**
(21) Anmeldenummer: 00907557.3
(22) Anmeldetag: 12.02.2000
(51) Int. Cl.: C07D 405/06, A61K 31/4748, A61P 25/08, A61P 25/14, A61P 25/18

(54) **SUBSTITUIERTE 1,2,3,4,5,6-HEXAHYDRO-2,6-METHANO-3-BENZAZOCIN-10-OLE, VERFAHREN ZU IHRER HERSTELLUNG UND IHRE VERWENDUNG ALS ARZNEIMITTEL**
SUBSTITUTED 1,2,3,4,5,6-HEXAHYDRO-2,6-METHANO-3-BENZAZOCIN-10-OLS, METHOD FOR PRODUCING THEM AND THEIR USE AS MEDICAMENTS
1,2,3,4,5,6-HEXAHYDRO-2,6-METHANO-3-BENZAZOCIN-10-OLS SUBSTITUES, LEUR PROCEDE DE PRODUCTION ET LEUR UTILISATION COMME MEDICAMENTS

(30) Priorität: 23.02.1999 DE 19907874
(43) Veröffentlichungstag der Anmeldung: 28.11.2001
(73) Patentinhaber: Boehringer Ingelheim Pharma KG, 55216 Ingelheim am Rhein (DE)
(72) Erfinder: GRAUERT, Matthias, D-55218 Ingelheim am Rhein (DE); BRIEM, Hans, D-55218 Ingelheim am Rhein (DE); HOFFMANN, Matthias, D-55218 Ingelheim am Rhein (DE); CARTER, Adrian, D-55411 Bingen am Rhein (DE); WEISER, Thomas, D-55268 Nieder-Olm (DE); BECHTEL, Wolf-Dietrich, D-55437 Appenheim (DE); PALLUK, Rainer, D-55411 Bingen am Rhein (DE)
(86) Internationale Anmeldenummer: EP0001160
(87) Internationale Veröffentlichungsnummer: WO00050421

(56) Entgegenhaltungen:
- EP-A- 0 370 499
- AU-B- 524 600

## Beschreibung

Die vorliegende Erfindung betrifft N-(5-Phenyl-tetrahydrofuranyl)methyl- und N-(6-Phenyl-tetrahydropyranyl)methyl- substituierte 1,2,3,4,5,6-Hexahydro-2,6-methano-3-benzazocin-10-ole der allgemeinen Formel **1**, Verfahren zu ihrer Herstellung und ihre Verwendung als Arzneimittel, 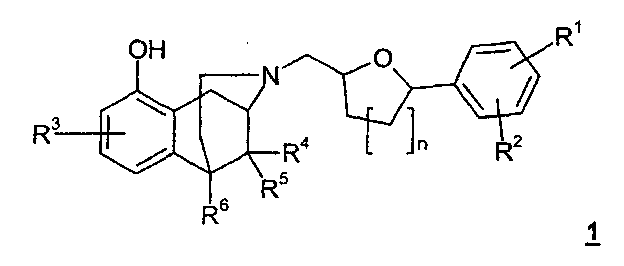 worin
- R¹: Wasserstoff, Methyl oder Fluor;
- R²: Wasserstoff, Methyl oder Fluor;
- n: eine ganze Zahl 1 oder 2;
- R³: Wasserstoff, Fluor, Chlor, Brom, Methyl, Ethyl, Hydroxy oder Methoxy;
- R⁴: Wasserstoff oder Methyl;
- R⁵: Wasserstoff oder Methyl;
- R⁶: Wasserstoff, Methyl oder Ethyl
bedeuten können.

Bevorzugt sind die Verbindungen der allgemeinen Formel **1**, in der
- R¹: Wasserstoff oder Fluor;
- R²: Wasserstoff oder Fluor;
- n: eine Zahl 1;
- R³: Wasserstoff oder Methyl;
- R⁴: Wasserstoff oder Methyl;
- R⁵: Wasserstoff oder Methyl;
- R⁶: Wasserstoff, Methyl oder Ethyl;
bedeuten können.

Gegenstand der Erfindung sind die jeweiligen Verbindungen gegebenenfalls in Form der einzelnen optischen Isomeren, Mischungen der einzelnen Enantiomeren oder Racemate sowie in Form der freien Basen oder der entsprechenden Säureadditionssalze mit pharmakologisch unbedenklichen Säuren - wie beispielsweise Säureadditionssalze mit Halogenwasserstoffsäuren - beispielsweise Chlor- oder Bromwasserstoffsäure - oder organische Säuren - wie z.B. Oxal-, Fumar- oder Diglycolsäure oder Methansulfonsäure.

EP-A-0 370 499 beschreibt die Verwendung ähnlicher Verbindungen, wie beispielsweise N-(Tetrahydrofuran-2-ylmethyl)-substituierte Hydroxybenzomorphane, als zytoprotektive Wirkstoffe zur Behandlung von Gehirnischämien, Epilepsie und neurodegenerativen Erkrankungen.

### Biologische Eigenschaften

Die beanspruchten Verbindungen sind Blocker des spannungsabhängigen Natriumkanals. Es handelt sich dabei um Verbindungen, die Batrachotoxin (BTX) mit hoher Affinität (Kᵢ < 1000 nM) kompetitiv oder nicht-kompetitiv von der Bindungsstelle am Natriumkanal verdrängen. Solche Substanzen zeigen eine "usedependency" bei der Blockade der Natriumkanäle, d.h. für die Bindung der Substanzen an dem Natriumkanal müssen die Natriumkanäle zunächst aktiviert werden. Die maximale Blockade der Natriumkanäle wird erst nach wiederholter Stimulation der Natriumkanäle erreicht. Demzufolge binden die Substanzen bevorzugt an Natriumkanäle, die vermehrt aktiviert werden. Dadurch sind die Substanzen in der Lage, bevorzugt in den Körperregionen wirksam zu werden, die pathologisch überstimuliert sind. Die erfindungsgemäßen Verbindungen der allgemeinen Formel **1** können somit bei Erkrankungen, deren Ursache auf eine durch Übererregung bedingte Funktionsstörung beruhen, eingesetzt werden. Darunter fallen Erkrankungen wie Arrhythmien, Spasmen, kardiale und Gehimischämien, Schmerzen sowie neurodegenerative Erkrankungen verschiedener Genese. Beispielsweise kann genannt werden: Epilepsie, Hypoglykämie, Hypoxie, Anoxie, Gehirntrauma, Gehirnoedem, Gehirn-Schlaganfall, perinatale Asphyxie, Degenerationen des Cerebellums, amyotrope laterale Sklerose, Morbus Huntington, Morbus Alzheimer, Morbus Parkinson, Zyklophrenie, Hypotonie, Herzinfakt, Herzrhythmusstörungen, Angina pectoris, chronischer Schmerz, neuropathischer Schmerz sowie Lokalanaesthesie.

Als Testsystem für den Nachweis der Natriumkanal blockierenden Wirkung dient die BTX-Bindung am Natriumkanal [S.W. Postma & W.A. Catterall, Mol. Pharmacol 25, 219-227 (1984)] sowie patch-clamp Experimente, in denen gezeigt werden kann, daß die erfindungsgemäßen Verbindungen den elektrisch stimulierten Natriumkanal in einer "use-dependent" Art und Weise blockieren [W.A. Catterall, Trends Pharmacol. Sci., 8, 57-65 (1987)]. Durch die Auswahl des Zellsystems (z.B. neuronale, kardiale, DRG Zellen) kann die Wirkung der Substanzen auf verschiedene Natriumkanalsubtypen untersucht werden.
Die Natriumkanal blockierende Eigenschaft der erfindungsgemäßen Verbindungen kann durch die Blockade des Veratridin induzierten Glutamat-releases nachgewiesen werden [S. Villauneva, P. Frenz, Y. Dragnic, F. Orrego, Brain Res. 461, 377-380 (1988)]. Veratridin ist ein Toxin, das den Natriumkanal permanent öffnet. Dadurch kommt es zu einem erhöhten Einstrom von Natriumionen in die Zelle. Über die oben beschriebene Kaskade führt dieser Natriumeinstrom in neuronalem Gewebe zu einer gesteigerten Freisetzung von Glutamat. Durch die erfindungsgemäßen Verbindungen läßt sich diese Glutamatfreisetzung antagonisieren.

Antikonvulsive Eigenschaften der erfindungsgemäßen Substanzen wurden durch eine protektive Wirkung gegen Krämpfe, die durch einen maximalen Elektroschock in Mäusen ausgelöst wurden, belegt [M. A. Rogawski & R.J. Porter, Pharmacol. Rev. 42, 223-286 (1990)].

Neuroprotective Eigenschaften wurden durch protektive Wirkung in einem Ratten-MCAO-Modell [U. Pschorn & A. J. Carter, J. Stroke Cerebrovascular Diseases, 6, 93-99 (1996)] sowie einem Malonat induziertem Läsionsmodel [M.F. Beal, Annals of Neurology, 38, 357-366 (1995) und J.B. Schulz, R.T. Matthews, D.R. Henshaw und M.F. Beal, Neuroscience, 71, 1043-1048 (1996)] belegt.
Analgetische Wirkungen lassen sich in Modellen der diabetischen Neuropathie sowie in einem Ligatur-Modell belegen [C. Courteix, M. Bardin, C. Chantelauze, J. Lavarenne, A. Eschalier, Pain 57, 153-160 (1994); C. Courteix, A. Eschalier, J. Lavarenne, Pain 53, 81-88 (1993); G. J. Bennett und Y.-K. Xie, Pain 33, 87-107 (1988)].
Ferner wurde beschrieben, daß Natriumkanalblocker zur Therapie der Zyklophrenie (manisch depressive Erkrankung) eingesetzt werden können [J. R. Calabrese, C. Bowden, M.J. Woyshville; in: Psychopharmacology: The Fourth Generation of Progress (Eds.: D. E. Bloom and D. J. Kupfer) 1099-1111. New York: Raven Press Ltd.].

### Herstellungsverfahren

Die beanspruchten Verbindungen **1** lassen sich nach an sich aus dem Stand der Technik bekannten Verfahren herstellen. Ein möglicher Syntheseweg ist in Schema 1 dargestellt. Die Synthesen der als Ausgangsverbindung benötigten Nor-1,2,3,4,5,6-Hexahydro-2,6-methano-3-benzazocin-10-ole (**2**) sind in den deutschen Offenlegungsschriften 41 21 821, 195 28 472 und 197 40 110 beschrieben. Der Synthesebaustein **3** enthält eine Abgangsgruppe X, die bevorzugt lod, Brom oder ein Methansulfonat-Rest ist. Die Synthese des 5-Phenyl-tetrahydrofuran-2-yl-methyliodids ist in der Literatur [K. Miura, T. Hondo, S. Okajima, A. Hosomi, Tetrahedron Lett. 37 (1996) 487-490] für das Racemat beschrieben. Analog können nach dieser Vorschrift die enantiomerenreinen Verbindungen dargestellt werden, wenn man von dem entsprechenden chiralen 5-Hydroxy-5-phenyl-penten ausgeht [vergl. D. Seebach, R.E. Marti, T. Hintermann; Helv. Chim. Acta, 79 (1996) 1710-1740]. - Die entsprechenden Bromide lassen sich in analoger Weise darstellen, wenn man Brom anstelle von lod verwendet.
Die Methansulfonate der 6-Phenyl-tertahydropyran-2-yl-methanole und 5-Phenyltetrahydrofuran-2-yl-methanole lassen sich aus den entsprechenden Alkoholen darstellen. Die Synthese von 6-Phenyl-tetrahydropyran-2-yl-methanol und 5-Phenyltetrahydrofuran-2-yl-methanol ist in der Literatur beschrieben [T. Mandai, M. Ueda, K. Kashiwagi, M. Kawada, J. Tsuji, Tetrahedron Lett., 34 (1993) 111-114; S. Inoki, T. Mukaiyama, Chemistry Lett. 1990, 67-70].

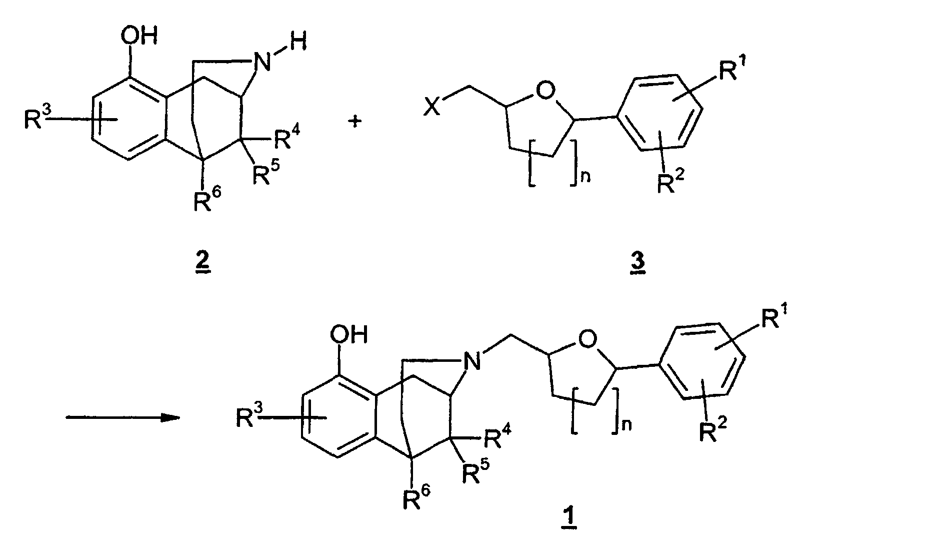

### Schema 1:

Die nachfolgenden Beispiele dienen der näheren Erläuterung der Erfindung, ohne diese jedoch auf die beispielhaft offenbarten Verbindungen und Verfahren zu beschränken.

### Beispiel 1: (2R, 5S)- und (2S, 5S)-5-Phenyl-tetrahvdrofuran-2-vl-methvl-bromid

1,6 g (10 mmol) (5S)-5-Hydroxy-5-phenyl-penten werden in 16 mL Dichlormethan gelöst und bei 10 bis 15 °C mit 1,6 g Brom in 16 mL Dichlormethan versetzt. Man läßt auf Raumtemperatur kommen und fügt 2 g Na₂CO₃ (Natriumcarbonat) und 0,1 g Tetrabutylammoniumsulfat zu. Nach 1 Stunde (h) werden 20 mL Wasser hinzugefügt und eine weitere Stunde bei Raumtemperatur gerührt. Die organische Phase wird abgetrennt, einmal mit 20 mL 2 N Salzsäure gewaschen, getrocknet und das Lösungsmittel im Vakuum abgezogen. Der Rückstand wird an 400 g Kieselgel (Cyclohexan/Essigester: 95 : 5) chromatographiert. Man erhält 0,6 g (25 % d. Th.) (2S, 5S)-5-Phenyl-tetrahydrofuran-2-yl-methyl-bromid und 0,7 g (29 % d. Th.) (2R, 5S)-5-Phenyl-tetrahydrofuran-2-yl-methyl-bromid.

### Beispiel 2: (2S, 5S)-Methansulfonsäure-(5-phenyl-tetrahydrofuran-2-yl)methvlester

580 mg (3,3 mmol) (2S, 5S)-5-Phenyl-tetrahydrofuran-2-yl-methanol werden in 4 mL Pyridin gelöst und mit 390 mg (3,4 mmol) Methansulfonsäurechlorid versetzt und 1 h bei 0 °C und anschließend 8 h bei Raumtemperatur gerührt. Anschließend versetzt man mit 30 mL Wasser und 30 mL 2 N Salzsäure. Man extrahiert dreimal mit je 20 mL Diethylether, wäscht die etherische Phase einmal mit 50 mL 10%iger Na₂CO₃-Lösung, trocknet und zieht das Lösungsmittel im Vakuum ab. Der Rückstand wird über 20 g Kieselgel (Cyclohexan/Essigester: 1 : 1) chromatographiert. Man erhält 450 mg (53% d. Th:) der Titelverbindung als Öl.

### Beispiel 3: (2R,6S,2R',5'S)-N-[5'-Phenyl-tetrahydrofuran-2'-yl)methyl]-1,2,3,4,5,6-hexahydro-6,11,11-trimethyl-2,6-methano-3-benzazocin-10-ol-hydrochlorid

0,5 g (2,15 mmol) (2R,6S)-1,2,3,4,5,6-Hexahydro-6,11,11-trimethyl-2,6-methano-3-benzazocin-10-ol und 0,5 g (11 mmol) (2R, 5S)-5-Phenyl-tetrahydrofuran-2-ylmethyl-bromid werden in 3 mL 1,3-Dimethyltetrahydropyrimidinon gelöst und mit 1 g K₂CO₃ (Kaliumcarbonat) versetzt. Man erhitzt über einen Zeitraum von 4 h auf eine temperatur in einem Intervall von 80-90 °C, läßt abkühlen, versetzt mit 100 mL Wasser und extrahiert zweimal mit je 100 mL Essigester (Essigsäureethylester). Die vereinigten organischen Extrakte werden dreimal mit 100 mL Wasser gewaschen, getrocknet und im Vakuum vom Lösungsmittel befreit. Der Rückstand wird über 30 g Kieselgel (Cyclohexan/Essigester) chromatographiert. Die geeigneten Fraktionen werden gesammelt, das Lösungsmittel im Vakuum abgezogen, der Rückstand in 50 mL Ether aufgenommen und mit etherischer Salzsäure das Hydrochlorid gefällt. Man erhält auf diese Weise 0,5 g (54% d. Th.) der Titelverbindung als Kristalle vom Schmp.: 174 °C, [α]_{D}²⁰ = (-) 47,0° (c=1 in Methanol).

In Analogie zu Beispiel 3 wurden u.a. ferner dargestellt:
(2R,6S,2S',5'S)-N-[5'-Phenyl-tetrahydrofuran-2'-yl)methyl]-1,2,3,4,5,6-hexahydro-6,11,11-trimethyl-2,6-methano-3-benzazocin-10-ol-hydrochlorid;
Schmp.: 253 °C, [α]_{D}²⁰ = (-) 55,3° (c=1 in Methanol).
(2R,6S,2R',5'R)-N-[5'-Phenyl-tetrahydrofuran-2'-yl)methyl]-1,2,3,4,5,6-hexahydro-6,11,11-trimethyl-2,6-methano-3-benzazocin-10-ol-hydrochlorid;
Schmp.: 157 °C und
(2R,6S,2S',5'R)-N-[5'-Phenyl-tetrahydrofuran-2'-yl)methyl]-1,2,3,4,5,6-hexahydro-6,11,11-trimethyl-2,6-methano-3-benzazocin-10-ol-hydrochlorid;
Schmp.: 169°C.
Es wurde (2R, 5R)- und (2S, 5R)-5-Phenyl-tetrahydrofuran-2-yl-methyl-iodid als Isomerengemisch eingesetzt und die entsprechenden Diastereomere wurden auf chromatographischem Wege getrennt.

### Beispiel 4: (2RS,6RS,2S',5'S)-N-[5'-Phenyl-tetrahydrofuran-2'-yl)methyl]-1,2,3,4,5,6-hexahydro-6,7-dimethvl-2,6-methano-3-benzazocin-10-ol-hydrochlorid

440 mg (1,7 mmol) (2RS,6RS)-1,2,3,4,5,6-Hexahydro-6,7-dimethyl-2,6-methano-3-benzazocin-10-ole und 350 mg (1,6 mmol) (2S, 5S)-Methansulfonsäure-(5-phenyltetrahydrofuran-2-yl)methylester werden in 3 mL 1,3-Dimethyltetrahydropyrimidinon gelöst und mit 1 g K₂CO₃ versetzt. Man erhitzt über einen Zeitraum von 5 h auf eine Temperatur in einem Bereich von 80-90 °C, läßt abkühlen, versetzt mit 100 mL Wasser und extrahiert zweimal mit je 100 mL Essigester. Die vereinigten organischen Phasen werden dreimal mit 100 mL Wasser gewaschen, getrocknet und im Vakuum eingeengt. Der Rückstand wird über 30 g Kieselgel (Cyclohexan/Essigester 3 : 1) chromatographiert. Die geeigneten Fraktionen werden gesammelt, das Lösungsmittel im Vakuum abgezogen, der Rückstand in 50 mL Ether aufgenommen und mit etherischer Salzsäure das Hydrochlorid gefällt. Ausbeute: 370 mg (53%) eines 1:1 Diastereomerengemisches, Schmp.: 155 °C.

### Pharmazeutische Zubereitungen

Im folgenden sind einige Beispiele für pharmazeutische Zubereitungen mit dem Wirkstoff angegeben:

### Tabletten:

| | |
|---|---|
| Wirkstoff gemäß allgemeiner Formel **1** | 20 mg |
| Magnesiumstearat | 1 mg |
| Laktose | 190 mg |

### Lösung zur Injektion

| | |
|---|---|
| Wirkstoff gemäß allgemeiner Formel **1** | 0,3 mg |
| Natriumchlorid | 0,8 g |
| Benzalkoniumchlorid | 0,01 mg |
| Aqua ad injectionem ad 100 ml | |

Eine ähnliche Lösung zu der oben aufgeführten ist geeignet für die nasale Applikation in einem Spray, oder in Kombination mit einem Gerät, das ein Aerosol mit einer Partikelgröße vorzugsweise zwischen 2 und 6 µM zur Anwendung über die Lunge generiert.

### Lösung zur Infusion

Eine 5 gewichtsprozentige Xylit- oder eine Kochsalzlösung, die beispielseise den Wirkstoff in einer Konzentration von 2 mg/ml enthält, wird mit einem Natriumacetat-Puffer auf einen pH-Wert von ca. 4 eingestellt.

Derartige Infusionslösungen können einen Gehalt an Wirkstoff gemäß der allgemeinen Formel **1** bezogen auf die Gesamtmasse der pharmazeutischen Zubereitung in einem Bereich von 0,001 bis 5 Gew.-%, vorzugsweise in einem Bereich von 0,001 bis 3 Gew.-% und besonders bevorzugt in einem Bereich von 0,01 bis 1 Gew.-% aufweisen.

### Kapseln für die Inhalation

Der Wirkstoff gemäß der allgemeinen Formel **1** wird in mikronisierter Form (Partikelgröße im wesentlichen zwischen 2 und 6 µM), gegebenfalls unter Zusatz von mikronisierten Trägersubstanzen, etwa Laktose, in Hartgelatinekapseln gefüllt. Zur Inhalation dienen übliche Geräte für die Pulverinhalation. In jede Kapsel werden z.B. zwischen 0,2 und 20 mg Wirkstoff und 0 bis 40 mg Laktose eingefüllt.

### Inhalationsaerosol

| | |
|---|---|
| Wirkstoff gemäß allgemeiner Formel **1** | 1 Teil |
| Sojalecithin | 0,2 Teile |
| Treibgasmischung ad 100 | |

## Patentansprüche

1. 1,2,3,4,5,6-Hexahydro-2,6-methano-3-benzazocin-10-ole der allgemeinen Formel **1** 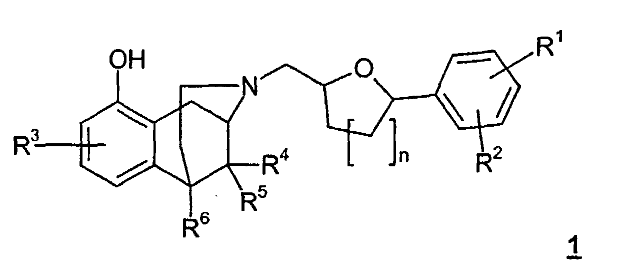 worin
R¹ Wasserstoff, Methyl oder Fluor;
R² Wasserstoff, Methyl oder Fluor;
n eine ganze Zahl 1 oder 2;
R³ Wasserstoff, Fluor, Chlor, Brom, Methyl, Ethyl, Hydroxy oder Methoxy;
R⁴ Wasserstoff oder Methyl;
R⁵ Wasserstoff oder Methyl;
R⁶ Wasserstoff, Methyl oder Ethyl
bedeuten können,
die jeweiligen Verbindungen gegebenenfalls in Form der einzelnen optischen Isomeren, Mischungen der einzelnen Enantiomeren oder Racemate sowie in Form der freien Basen oder der entsprechenden Säureadditionssalze mit pharmakologisch unbedenklichen Säuren.

2. Verbindungen der allgemeinen Formel 1 gemäß Anspruch 1, worin
R¹ Wasserstoff oder Fluor;
R² Wasserstoff oder Fluor;
n eine Zahl 1;
R³ Wasserstoff oder Methyl;
R⁴ Wasserstoff oder Methyl;
R⁵ Wasserstoff oder Methyl;
R⁶ Wasserstoff, Methyl oder Ethyl
bedeuten können,
die jeweiligen Verbindungen gegebenenfalls in Form der einzelnen optischen Isomeren, Mischungen der einzelnen Enantiomeren oder Racemate sowie in Form der freien Basen oder der entsprechenden Säureadditionssaize mit pharmakologisch unbedenklichen Säuren.

3. Verfahren zur Herstellung von Verbindungen der allgemeinen Formel 1 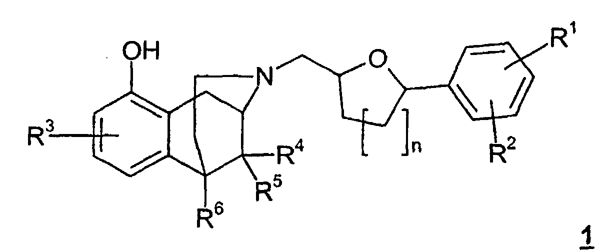 **dadurch gekennzeichnet, daß** man ein Norbenzomorphanderivat der allgemeinen Formel **2** 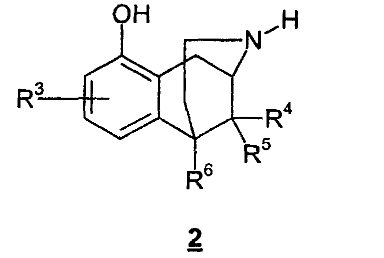 worin R³, R⁴, R⁵ und R⁶ die in den Ansprüchen 1 oder 2 genannte Bedeutung haben können, mit einem Tetrahydrofuran- bzw. Hexahydropyranderivat der allgemeinen Formel **3** 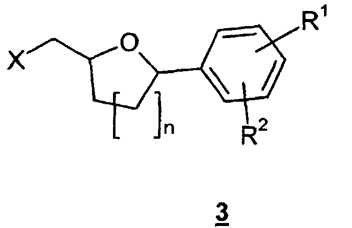 in der R¹, R² und n die in den Ansprüchen 1 oder 2 genannte Bedeutung haben können und X eine gegen eine sekundäre Aminogruppe substituierbare Austrittsgruppe verkörpert, umsetzt und das Endprodukt gegebenenfalls reinigt und isoliert.

4. Pharmazeutische Zubereitung **gekennzeichnet durch** einen Gehalt an einer der Verbindungen nach einem der Ansprüche 1 oder 2 und deren Säureadditionssalze neben üblichen Hilfs- und Trägerstoffen.

5. Pharmazeutische Zubereitung nach Anspruch 4, **dadurch gekennzeichnet, daß** sie als Infusionslösung formuliert ist.

6. Pharmazeutische Zubereitung nach Anspruch 5, **dadurch gekennzeichnet, daß** der Wirkstoffgehalt bezogen auf die Gesamtmasse der pharmazeutischen Zubereitung in einem Bereich von 0,001 bis 5 Gew.-% liegt.

7. Pharmazeutische Zubereitung nach Anspruch 6, **dadurch gekennzeichnet, daß** der Wirkstoffgehalt bezogen auf die Gesamtmasse der pharmazeutischen Zubereitung in einem Bereich von 0,001 bis 3 Gew.-% liegt.

8. Pharmazeutische Zubereitung nach Anspruch 7, **dadurch gekennzeichnet, daß** der Wirkstoffgehalt bezogen auf die Gesamtmasse der pharmazeutischen Zubereitung in einem Bereich von 0,01 bis 1 Gew.-% liegt.

9. Verwendung von Verbindungen nach einem der Ansprüche 1 oder 2 als Arzneimittel.

10. Verwendung von Verbindungen nach einem der Ansprüche 1 oder 2 zur Herstellung eines Arzneimittels zur therapeutischen Behandlung von durch Übererregung bedingten Funktionsstörungen.

11. Verwendung von Verbindungen nach einem der Ansprüche 1 oder 2 zur Herstellung eines Arzneimittels zur therapeutischen Behandlung von Arrhythmien, Spasmen, kardialen und Gehirnischämien, Schmerzen sowie neurodegenerativen Erkrankungen verschiedener Genese.

12. Verwendung von Verbindungen nach Anspruch 11 zur Herstellung eines Arzneimittels zur therapeutischen Behandlung von Epilepsie, Hypoglykämie, Hypoxie, Anoxie, Gehimtrauma, Gehirnoedem, Gehirn-Schlaganfall, perinatale Asphyxie, Degenerationen des Cerebellums, amyotrope laterale Sklerose, Morbus Huntington, Morbus Alzheimer, Morbus Parkinson, Zyklophrenie, Hypotonie, Herzinfakt, Herzrhythmusstörungen, Angina pectoris, chronischem Schmerz, neuropathischer Schmerz sowie Lokalanaesthesie.

## Claims

1. 1,2,3,4,5,6-Hexahydro-2,6-methano-3-benzazocin-10-ols of general formula **1** 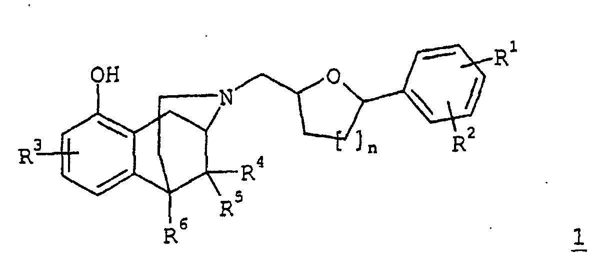 wherein
R₁ may denote hydrogen, methyl or fluorine;
R₂ may denote hydrogen, methyl or fluorine;
n may denote an integer 1 or 2;
R₃ may denote hydrogen, fluorine, chlorine, bromine, methyl, ethyl, hydroxy or methoxy;
R₄ may denote hydrogen or methyl;
R₅ may denote hydrogen or methyl;
R₆ may denote hydrogen, methyl or ethyl,
the compounds in question optionally in the form of the individual optical isomers, mixtures of the individual enantiomers or racemates and in the form of the free bases or the corresponding acid addition salts with pharmacologically acceptable acids.

2. Compounds of general formula **1** according to claim 1, wherein
R₁ may denote hydrogen or fluorine;
R₂ may denote hydrogen or fluorine;
n may denote the number 1;
R₃ may denote hydrogen or methyl;
R₄ may denote hydrogen or methyl;
R₅ may denote hydrogen or methyl;
R₆ may denote hydrogen, methyl or ethyl
the compounds in question optionally in the form of the individual optical isomers, mixtures of the individual enantiomers or racemates and in the form of the free bases or the corresponding acid addition salts with pharmacologically acceptable acids.

3. Process for preparing compounds of general formula 1 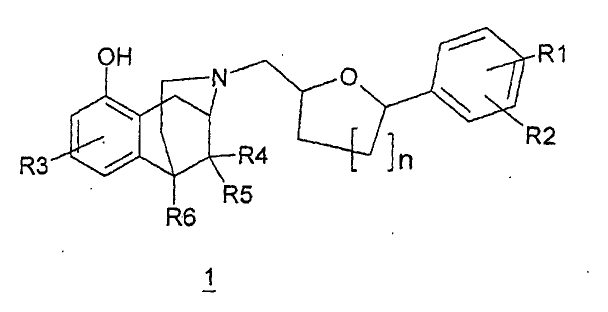 **characterised in that** a norbenzomorphane derivative of general formula **2** 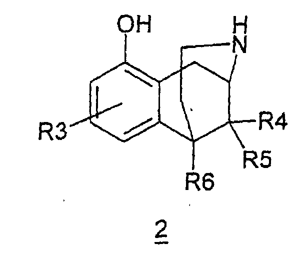 wherein R₃, R₄, R₅ and R₆ are defined as in claim 1 or 2, is reacted with a tetrahydrofuran or hexahydropyran derivative of general formula **3** 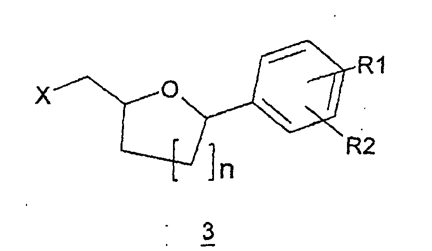 wherein R₁, R₂ and n are defined as in claim 1 or 2 and X represents a leaving group which can be substituted by a secondary amino group and the end product is optionally purified and isolated.

4. Pharmaceutical preparation, **characterised in that** it contains one of the compounds according to one of claims 1 or 2 and the acid addition salts thereof together with conventional excipients and carriers.

5. Pharmaceutical preparation according to claim 4, **characterised in that** it is formulated as a solution for infusion.

6. Pharmaceutical preparation according to claim 5, **characterised in that** the content of active substance is within the range from 0.001 to 5 wt.%, based on the total mass of the pharmaceutical preparation.

7. Pharmaceutical preparation according to claim 6, **characterised in that** the content of active substance is within the range from 0.001 to 3 wt.%, based on the total mass of the pharmaceutical preparation.

8. Pharmaceutical preparation according to claim 7, **characterised in that** the content of active substance is within the range from 0.01 to 1 wt.%, based on the total mass of the pharmaceutical preparation.

9. Use of compounds according to one of claims 1 and 2 as pharmaceutical compositions.

10. Use of compounds according to one of claims 1 and 2 for preparing a pharmaceutical composition for the therapeutic treatment of functional disorders caused by overstimulation.

11. Use of compounds according to one of claims 1 and 2 for preparing a pharmaceutical composition for the therapeutic treatment of arrhythmias, spasms, cardiac and cerebral ischaemia, pain and neurodegenerative disorders of various origins.

12. Use of compounds according to claim 11 for preparing a pharmaceutical composition for the therapeutic treatment of epilepsy, hypoglycaemia, hypoxia, anoxia, brain trauma, brain oedema, cerebral stroke, perinatal asphyxia, degeneration of the cerebellum, amyotropic lateral sclerosis, Huntington's disease, Alzheimer's disease, Parkinson's disease, cyclophrenia, hypotonia, cardiac infarct, heart rhythm disorders, angina pectoris, chronic pain, neuropathic pain and local anaesthesia.

## Revendications

1. 1,2,3,4,5,6-hexahydro-2,6-méthano-3-benzazocin-10-ols de formule générale 1 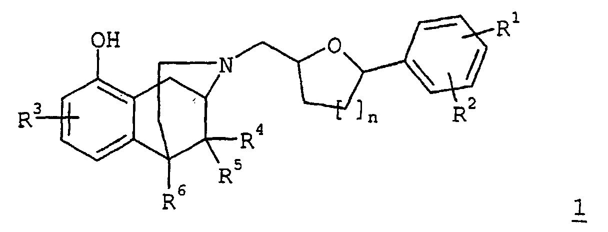 où
R¹ peut représenter l'hydrogène, méthyle ou le fluor ;
R² peut représenter l'hydrogène, méthyle ou le fluor ;
n peut représenter un nombre entier 1 ou 2 ;
R³ peut représenter l'hydrogène, le fluor, le chlore, le brome, méthyle, éthyle, hydroxyle ou méthoxy ;
R⁴ peut représenter l'hydrogène ou méthyle ;
R⁵ peut représenter l'hydrogène ou méthyle ;
R⁶ peut représenter l'hydrogène, méthyle ou éthyle ;
les différents composés éventuellement sous forme des isomères optiques individuels, de mélanges des énantiomères individuels ou de racémates ainsi que sous forme des bases libres ou des sels d'addition d'acide correspondants avec des acides pharmacologiquement acceptables.

2. composés de formule générale 1 selon la revendication 1 où
R¹ peut représenter l'hydrogène ou le fluor ;
R² peut représenter l'hydrogène ou le fluor ;
n peut représenter un nombre 1 ;
R³ peut représenter l'hydrogène ou méthyle ;
R⁴ peut représenter l'hydrogène ou méthyle ;
R⁵ peut représenter l'hydrogène ou méthyle ;
R⁶ peut représenter l'hydrogène, méthyle ou éthyle ;
les différents composés éventuellement sous forme des isomères optiques individuels, de mélanges des énantiomères individuels ou de racémates ainsi que sous forme des bases libres ou des sels d'addition d'acide correspondants avec des acides pharmacologiquement acceptables.

3. Procédé de préparation de composés de formule générale 1 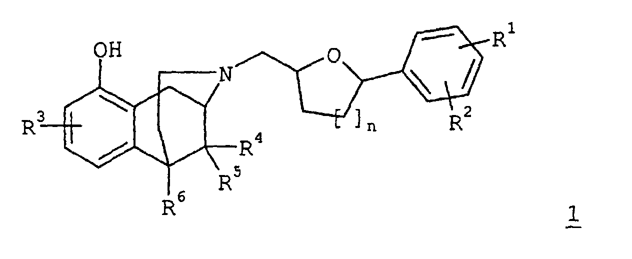 **caractérisé en ce que** l'on fait réagir un dérivé de norbenzomorphane de formule générale 2 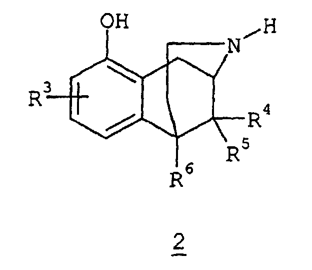 où R³, R⁴, R⁵ et R⁶ peuvent avoir la signification indiquée dans les revendications 1 et 2, avec un dérivé de tétrahydrofurane ou d'hexahydropyrane de formule générale 3 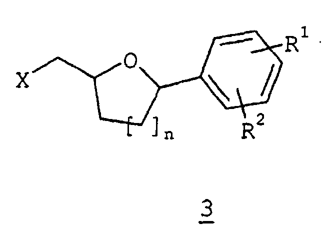 où R¹, R² et n peuvent avoir la signification indiquée dans les revendications 1 et 2 et X représente un groupe partant remplaçable par un groupe amino secondaire et, éventuellement, on purifie et isole le produit final.

4. Préparation pharmaceutique **caractérisée par** une teneur en l'un des composés selon l'une des revendications 1 et 2 et de ses sels d'addition d'acide, outre des adjuvants et supports habituels.

5. Préparation pharmaceutique selon la revendication 4 **caractérisée en ce qu'**elle est formulée sous forme de solution pour perfusion.

6. Préparation pharmaceutique selon la revendication 5 **caractérisée en ce que** la teneur en principe actif par rapport à la masse totale de la préparation pharmaceutique est située dans un domaine de 0,001 à 5 % en masse.

7. Préparation pharmaceutique selon la revendication 6 **caractérisée en ce que** la teneur en principe actif par rapport à la masse totale de la préparation pharmaceutique est située dans un domaine de 0,001 à 3 % en masse.

8. Préparation pharmaceutique selon la revendication 7 **caractérisée en ce que** la teneur en principe actif par rapport à la masse totale de la préparation pharmaceutique est située dans un domaine de 0,01 à 1 % en masse.

9. Utilisation de composés selon l'une des revendications 1 et 2 comme médicament.

10. Utilisation de composés selon l'une des revendications 1 et 2 pour la production d'un médicament pour le traitement thérapeutique de troubles fonctionnels dus à une surexcitation.

11. Utilisation de composés selon l'une des revendications 1 et 2 pour la production d'un médicament pour le traitement thérapeutique d'arythmies, de spasmes, d'ischémies cardiaques et cérébrales, de douleurs ainsi que de maladies neurodégénératives de genèse diverse.

12. Utilisation de composés selon la revendication 11 pour la production d'un médicament pour le traitement thérapeutique de l'épilepsie, de l'hypoglycémie, de l'hypoxie, de l'anoxie, des traumatismes cérébraux, de l'oedème cérébral, de l'attaque cérébrale, de l'asphyxie périnatale, des dégénérescences du cervelet, de la sclérose latérale amyotrophique, de la maladie de Huntington, de la maladie d'Alzheimer, de la maladie de Parkinson, de la cyclothymie, de l'hypotonie, de l'infarctus du myocarde, des troubles du rythme cardiaque, de l'angine de poitrine, de la douleur chronique, de la douleur neuropathique ainsi que de l'anesthésie locale.
